(19) Europäisches Patentamt
European Patent Office
Office européen des brevets

(11) **EP 4 621 795 A1**

(12) **EUROPEAN PATENT APPLICATION**

(43) Date of publication:
**24.09.2025 Bulletin 2025/39**

(21) Application number: **24164662.9**

(22) Date of filing: **19.03.2024**

(51) International Patent Classification (IPC):
*G16H 40/20* (2018.01)    *G16H 10/40* (2018.01)

(52) Cooperative Patent Classification (CPC):
**G16H 10/40; G16H 40/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Roche Diagnostics GmbH
68305 Mannheim (DE)**
• **Roche Diagnostics International AG
6343 Rotkreuz (CH)**
• **F. Hoffmann-La Roche AG
4070 Basel (CH)**

(72) Inventors:
• **EZZI, Afshin
6343 Rotkreuz ZG (CH)**

• **GUTMANN, Oliver
6343 Rotkreuz ZG (CH)**
• **JAGDHUBER, Rudolf
82377 Penzberg (DE)**
• **KING, Michael
82377 Penzberg (DE)**
• **MENARD, Jeffrey Drew
6343 Rotkreuz ZG (CH)**
• **SCHWAB, Wolfgang Leonhard
4070 Basel BS (CH)**

(74) Representative: **Mewburn Ellis LLP
Aurora Building
Counterslip
Bristol BS1 6BX (GB)**

Remarks:
Amended claims in accordance with Rule 137(2) EPC.

(54) **COMPUTER-IMPLEMENTED METHOD FOR PROCESSING A REQUEST FOR AN ANALYTICAL TEST**

(57)    Provided is a computer-implemented method, the method comprising: receiving a request for an analytical test, the request defining one or more analytical tests to be performed on a biological sample; estimating, for each of a plurality of laboratories, a respective total time, $T_{total}$, to process the biological sample by the respective laboratory, and performing a routing action related to the biological sample on the basis of the respective total times. Estimating the total time is performed using a model which includes one or more complexity factors indicating a complexity of one or more analytical tests to be performed by the respective laboratory, the complexity factors being derived from historical testing data.

Fig. 2

**EP 4 621 795 A1**

# EP 4 621 795 A1

**Description**

## *TECHNICAL FIELD*

The present disclosure relates to methods and systems for processing a request for an analytical test.

## *BACKGROUND*

The increasingly aging population is leading to an increased demand on healthcare systems, including on laboratories for testing biological samples for diagnosing heath conditions. Increased demand on laboratories results higher testing volumes, higher costs, and higher resource usage involved in the running of such laboratories.

In large laboratory networks comprising a plurality of laboratories, there may be no access to the real-time workload and capacity of each laboratory which may lead to overloading of some laboratories while other libraries in the network may have available capacity which is not being utilised. Accordingly, uneven distribution of samples within a laboratory network can lead to delays in receiving test results leading to worse patient outcomes and increased costs for the healthcare system.

In telemedicine, in particular, wherein a virtual caregiver prescribes an analytical test to a patient, the virtual caregiver may instruct the patient to provide a sample to a laboratory for tests and book a follow-up appointment to discuss the results. However, virtual caregivers typically do not have access to laboratory workloads or sample processing wait times. Therefore, if the patient provides the sample to a laboratory which does not have capacity, the test results may be delayed and may not be ready for the follow-up appointment.

Accordingly, there is a need to improve the distribution of samples and the throughput of laboratories in a network in order to meet increased demand. The present disclosure was arrived at in light of the above considerations.

## *SUMMARY*

Broadly, the present invention relates to systems and methods for allocating biological samples to recommended laboratories based on an estimated total time to begin processing and process the sample in each of the laboratories. In particualer, the total time to process the sample is estimated using one or more complexity factors indicative of the complexity of analytical tests being performed by the respective laboratory. By accounting for the complexity of the tests, an improved estimate of laboratory wait times and processing times can be determined, enabling more informed decisions on laboratory allocation to be made.

Accordingly, in a first aspect of the present invention there is provided a computer-implemented method. The method comprises: receiving a request for an analytical test, the request defining one or more analytical tests to be performed on a biological sample; estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and performing a routing action related to the biological sample on the basis of the respective total times.

Advantageously, by using a complexity factor to determine the respective total times, a more accurate estimate of processing time for each lab can be determined. For example, the complexity factor may help the model to account for any increased risk of errors, additional processing steps, lab set-up time, additional staff training, etc which may be associated with more complex analytical tests based on the historical testing date. Further, by the improved prediction of total processing time can lead to improved distribution of samples across a laboratory network thereby increasing throughput and reducing delays in the laboratory network.

The invention includes the combination of the aspects and preferred features described except where such a combination is clearly impermissible or expressly avoided.

The computer-implemented method may be described as a method of processing a request for an analytical test, a method for allocating a biological sample to a laboratory for testing, or a method of estimating a total time $T_{total}$ for a biological sample to be processed by one or more laboratories. In particular, the computer-implemented method may form part of a computer implemented method for determining a distribution of biological samples across a plurality of laboratories for processing.

The request may be received from a caregiver such as a physician, for example, as part of a telemedicine consultation. As such, the biological sample may include one or more biological samples from a patient. The analytical tests may be a specification of one or more procedures or routines for processing the samples in order to obtain a result e.g., indicative of a state of health of the patient or a composition of the sample. The analytical tests may be performed by in-vitro diagnostic laboratory instruments, for example pre-analytical instruments, analytical instruments, post-analytical instruments, or instruments used in the transportation of the medical samples. For example, each laboratory may be an in-vitro diagnostic laboratory including at least one analytical instrument.

The respective total time $T_{total}$ may be a total laboratory throughput time of the sample based on the requested analytical tests associated with that sample. For example, the respective total time $T_{total}$ may include one or more of: a sample transport time to the laboratory, a sample wait time associated with a pending workload of the laboratory (i.e., a pending samples processing time of the laboratory), and/or a sample processing time associated with the requested analytical tests for the biological sample. Each of these estimated times may be determined according to the techniques discussed herein.

The complexity factors of the one or more analytical tests to be performed by the respective laboratory may be associated with one or more pending analytical tests of the laboratory and/or the one or more requested analytical tests of the specified biological sample. The complexity factor may be indicative of a prediction of analytical test complexity based on the historical testing data. The complexity factor may therefore provide a buffer factor enabling more complex analytical tested to be accounted for in the estimate of total time without causing the laboratory to run behind schedule if more complex analytical tests are ordered.

Using the model to estimate the respective total time, $T_{total}$, for each laboratory may include estimating, for each of a plurality of pending biological samples, a pending sample processing time, $SPT$. The pending biological samples may be samples assigned to or associated with the respective laboratory for performing one or more analytical tests. In particualer, the pending biological samples may include: samples actively being processed by the laboratory; samples queued to be processed by the laboratory; and /or samples announced and due for delivery to the laboratory.

The pending sample processing time, $SPT$, may be indicative of a predicted time for the pending biological sample to be tested. Accordingly, the pending sample processing time, $SPT$, may be determined according to one or more analytical tests specified to be performed on the respective pending sample. As such, the sample processing time may also be referred to as a request processing time, each request having one or more associated biological samples and one or more analytical tests for performing on the biological sample.

Each pending sample processing time, $SPT$, may be determined on the basis of the one or more complexity factors, the one or more complexity factors being determined using historical testing data. Each complexity factor may be associated with a particular analytical test. A complexity factor may be provided for each analytical test which is common to each laboratory. However, in other examples, a complexity factor for each analytical test may be determined for each laboratory (or for a type or a group of laboratories).

The sample processing time, $SPT$, for a given pending sample, $i$, may be estimated using the model, the model including the one or more complexity factors.

For example, the model may be a linear regression model of the form:

$$SPT_i = u_o + u_1 T_{i1} + u_2 T_{i2} + \cdots = \sum_{j}^{p} u_j T_{ij}$$

where $u_j$ is a complexity factors corresponding to analytical test $j$ to be performed on the (pending) sample, the variable $T_{ij}$ is either 0 or 1 dependant on whether the test $n$ is to be performed on the sample $i$ (e.g., as specified in an associated request), and $p$ is the total number of tests to be performed on the respective pending sample, the complexity factors $u_j$ being determined based on the historical testing data. $u_0$ may be a baseline value and $T_{10}$ (i.e., for i=1 and j=0) may be equal to 0. Accordingly, the contributions from individual specified tests, $T_{ij}$, may be accounted for in determining the total sample processing time, $SPT$, for each pending sample. In this way, a bespoke formula may be determined for each combination of tests requested for each sample.

The complexity factor for a given test, $u$, can be calculated via an ordinary least squares algorithm much like the regression coefficients of a linear regression model. The data used is that for historical $SPTs$, and for each historical $SPT$ the associated sets of tests $T_j$ is known. The values of $u$ typically do not depend on the individual sample $i$. The complexity factor may have the units of time, and may, for example be indicative of an averaged or mean sample processing time derived from historical data.

In some examples, the sample processing time may also be determined based on an estimation of interaction effects of multiple analytical tests interacting with each other to affect the sample processing time. For example, the interaction effects may be included in the estimation of $SPT_i$ as a linear combination, e.g., according to: $(\ldots + u_{1,2} T_1 T_2 + \ldots)$.

In some examples, the model may be a generalised linear model of the form:

$$E(SPT) = \mu = h(\mu_0 + \mu_1 T_1 + \cdots)$$

Estimating the respective total time, $T_{total}$, for each laboratory may include estimating a total pending samples processing time, $W$, on the basis of the pending sample processing times, $SPT$. The total pending samples processing time, $W$ may therefore be described as the predicted time taken by the respective laboratory to process each of the currently pending samples.

Accordingly, the total pending samples processing time, *W,* may also be referred to as a total waiting time or a queuing time associated with the respective laboratory. The total waiting time may represent a predicted time for which the biological sample must wait before the one or more analytical tests may begin at the respective laboratory.

Estimating the total pending samples processing time, *W,* may include determining whether any of the analytical tests for the pending samples can be performed in parallel (i.e., multiple tests being performed on a portion of a pending sample in parallel e.g., using aliquots). The calculation of the total pending samples processing time, *W,* may be adjusted based on this determination. In further examples, estimating the total pending samples processing time, *W,* may include determining whether any of the analytical tests can be performed on a plurality of the pending samples in parallel.

In some examples, the method may include determining a maximum waiting time, *Wmax,* the maximum waiting time being an upper boundary for the total samples processing time, wherein it is assumed that there is no parallelising (of biological samples and/or analytical tests) by the laboratory. *Wmax* may be determined according to:

$$W_{max} = \sum_{i=1}^{n} SPT_i$$

In further examples, a minimum waiting time, *Wmin*, representing a lower boundary for the total samples processing time, wherein it is assumed that the laboratory performed an optimum amount of parallelising (of biological samples and/or analytical tests). *Wmin* may be determined according to:

$$W_{min} = nu_0 + \frac{\sum_{i=1}^{n} u_1 T_{i1}}{\# \ Parallel \ 1} + \frac{\sum_{i=1}^{n} u_2 T_{i2}}{\# \ Parallel \ 2} + \dots$$

Where *#ParallelN* is the number of tests which could be performed in parallel. This factor is assigned to a test j, and can represent the number of multiple machines performing test j in parallel or any other way in which the load of samples which need test j can be split and performed at the same time.

The total time, $T_{total}$, may be determined on the basis of *Wmin,*. In this way, the library selection may be determined based on the minimum time that the laboratory may take to begin processing the biological sample. The present inventors have found that by comparing respective total times $T_{total}$ which are determined based on *Wmin* is a more suitable measurement for comparing laboratory processing times because it accounts for a laboratories ability to process samples in parallel. In other examples, the total time, $T_{total}$, may be determined on the basis of *Wmax*. This may be useful if a sample must be processed by a specified deadline, and so certainty may be given that the sample will be processed in time, even in a worst case scenario.

Estimating the respective total times $T_{total}$ may comprise estimating a time taken for the biological sample specified in the request to arrive at the respective laboratory, $T_{transport}$ (i.e., a "travel time0 or a "transport time"). The travel time may be determined based on a location of the respective laboratory and a source location of the request. In some examples, the travel time may be determined using static data, such as historical distribution data (which may take into account a time or day of the sample provision), and/or information from a real time navigation system (e.g., of a delivery vehicle)

Additionally, estimating the respective total times $T_{total}$ may comprise estimating a lab processing time taken to process the biological sample (specified in the request) at that respective laboratory $W_{package}$. The lab processing time may be a time taken for the respective laboratory to perform the one or more analytical tests specified in the analytical sample. The lab processing time may begin after the waiting time (i.e., the pending samples processing time) has expired.

The lab processing time $W_{package}$ may be determined on the basis of one or more sample processing times, *SPT,* associated with the biological sample and the one of the analytical tests specified in the request. The sample processing times, *SPT,* for the biological sample may be determined as described above for the pending biological samples.

The method may comprise determining a maximum lab processing time, $W_{max,package}$, in which it is assumed that no parallelisation of the one or more analytical tests is performed by the respective laboratory. The respective total time $T_{total}$ may therefore be estimated using $W_{max,package}$.

Additionally, the method may comprise determining a minimum lab processing time $W_{min,package}$ wherein it is assumed that the laboratory performs an optimum amount of the parallelisation of the one or more analytical tests. $W_{min,package}$ may be determined as described above for $W_{max}$.

The method may further include providing the estimated respective total times $T_{total}$, for each of the plurality of laboratories on a dashboard. For example, the dashboard may be a display or an information page of a web-application. The method may then comprise receiving an input indicative of a selected laboratory based on the total times, $T_{total}$, provided on the dashboard. The routing action may then comprise assigning the request to the selected laboratory.

The routing action may comprise routing or assigning the biological sample (or the request) to a selected laboratory of the plurality of laboratories, wherein the laboratory is selected on the basis of the plurality of estimated total times. For example, the selected laboratory may by the laboratory having a shortest respective total time, $T_{total}$.

In some examples, the request may also specify one or more laboratories at which the analytical test can be performed. For example, these may be default laboratories associated with a particular medical centre, care provide, or the one or more analytical tests specific in the request. The laboratory may be further selected on the basis of the specified laboratories. For example, the laboratory of the specified laboratories having the shortest respective total time, $T_{total}$ may be selected (i.e., to route the sample to).

In some examples, the request may also specify a desired time to result, DTR, for receiving results of the one or more analytical tests. Therefore, the routing action may comprise selecting a laboratory from a list of laboratories which have a total time, $T_{total}$, which fulfils the desired time to result.

In other examples, the request may include a specified laboratory (for example a predefined default laboratory e.g., selected according to cost and/or location). The method may then comprise comparing the DTR with the respective total time, $T_{total}$, for the specified laboratory, and, if the respective total time, $T_{total}$, is longer than the desired time to result, determining an alternative laboratory having a $T_{total}$, shorter than the DTR. In this example, the method may comprise, first estimating a respective total time $T_{total}$, for the laboratory specified in the request, and, if the respective total time $T_{total}$ is longer than the DTR, estimating a respective total time $T_{total}$ for one or more alternative laboratories. Selecting the alternative laboratory may comprise determining a laboratory having the shorted respective total time $T_{total}$. The method may comprise communicating the alternative laboratory to a user (e.g., by providing it to a dashboard. Further, the routing action may comprise routing the sample to the alternative laboratory (e.g., by assigning the request to the alternative laboratory).

The routing action may comprise recommending to a patient one or more medical centres or one or more of the plurality of laboratories to visit for sample collection. The or each medical centre may be associated with one or more of the plurality of laboratories. Recommending the medical centre to visit may include determining, from an electronic health record, a time of a follow-up medical appointment and recommending the one or more medical centres, or laboratories, where the total time, $T_{total}$, of the or each laboratory associated with the respective medical centre indicates the sample will be processed ahead of the follow-up medical appointment.

In some examples, the computer-implemented method may comprise received real-time (i.e., live) laboratory data and updating the one or more respective total times, $T_{total}$, based on the real-time data. The method may therefore include updating the routing action related to the biological sample on the basis of the updated total times. The real-time laboratory data may include information on laboratory malfunctions, laboratory closure days, updated complexity factors etc.

In a second aspect of the present invention there is provided a computer, comprising a processor and memory, the memory containing machine-executable instructions which when executed on the processor cause the processor to: receive a request for an analytical test, the request defining one or more analytical tests to be performed on a sample; estimate, for at least one of plurality of laboratories, a respective total time, $T_{total}$, to process the sample by the respective laboratory, wherein estimating the respective total time is performed using a model which includes one or more complexity factors based on the complexity of one or more analytical tests to be performed by the respective laboratory, the complexity factor being derived from historical testing data; and perform a routing action related to the biological sample on the basis of the respective total times.

In a third aspect, there is provided a system for allocating biological samples to laboratories for analytical tests, the system comprising: a request processing module configured to receive a request for an analytical test, the request defining one or more analytical tests to be performed on a biological sample; a laboratory capacity analysis module configured to estimate for each of a plurality of laboratories, a respective total time, $T_{total}$, to process the analytical tests to be performed on the biological sample by the respective laboratory, wherein estimating the total time is performed using a model which includes a complexity factor based on the complexity of the one or more analytical tests to be performed, the complexity factor being derived from historical testing data; a sample distribution module configured to determine a routing action related to the biological sample on the basis of the respective total times.

The request processing module, the laboratory capacity analysis module, and the sample distribution module configured may each be cloud-based. For example, the sample distribution module may form part of a web application for allocating samples to laboratories and communicating the allocation to patients.

The computer of the second aspect and the system of the third aspect may be configured to perform any one, or any combination insofar as they are compatible, of the optional features set out with reference to the first aspect.

Further aspects of the present invention provide: a computer program comprising code which, when run on a computer, causes the computer to perform the method of the first aspect; and a computer readable medium storing a computer program comprising code which, when run on a computer, causes the computer to perform the method of the first aspect.

**BRIEF DESCRIPTION OF THE DRAWINGS**

**Figure 1** is a flow diagram of a computer-implemented method according to an aspect of the present disclosure.

**Figure 2** is an example of a system according to an aspect of the present disclosure.

**Figure 3** is an example of a dashboard.

**Figure 4** is a further view of the dashboard of Figure 3.

**DETAILED DESCRIPTION**

Aspects and embodiments of the present invention will now be discussed with reference to the accompanying figures. Further aspects and embodiments will be apparent to those skilled in the art.

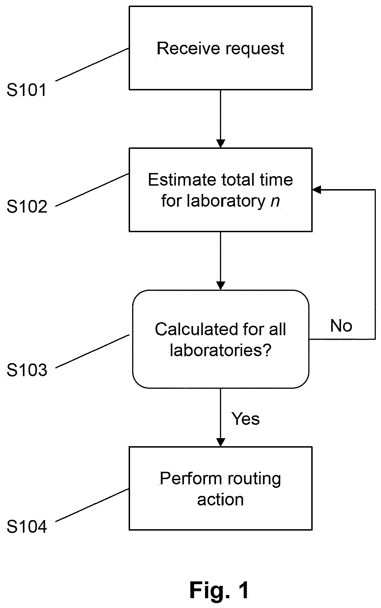

Figure 1 is a flow diagram of a computer-implemented method according to an aspect of the present disclosure. In a first step, S101, a request is received. The request defines one or more analytical tests to be performed on a biological sample. Next, in step S102, the total time $T_{total}$ to process the biological sample for laboratory $n$ is estimated. This is done by using a model which includes one or more complexity factors indicating a complexity of one or more analytical tests to be performed by the respective laboratory, the complexity factors being derived from historical testing data.

For example, every test which a laboratory is capable of performing is assigned a certain general complexity. These are defined with custom factors: $u_j$. These factors are fitted using historical data from each laboratory, as each laboratory has a large amount of data on sample processing times (SPT). An SPT is the time a sample needs from start to finish to perform all of its requested tests, and each sample comprises a certain combination of tests. From this historical data, a (generalised) linear regression model can be fitted of the form:

$$(SPT)_i = u_0 + u_1 T_{i1} + u_2 T_{i2} + \cdots = \Sigma_{j=1}^{p} u_j T_{ij} \quad \text{with } T_{10} = 1$$

to get the contributions of individual tests $T_{ij}$ for a given sample i on the total SPT. Here, $u_0$ is the baseline (non-test-specific) SPT portion, and $u_j$ is a measure of the complexity of test $j$. The variable $T_{ij}$ is either 0 or 1, indicating whether test $j$ was performed according to the sample request for sample $i$ or not.

In one example, the following complexity factors were derived from two laboratories for the following tests, using historical data for each of the tests as discussed above:

*Table 1*

| Test | Laboratory 1 $u_j$ values | Laboratory 2 $u_j$ values |
|---|---|---|
| Anti-HBe | 18 minutes | 20 minutes |
| Calcitonin | 19 minutes | 18 minutes |
| Troponin T | 9 minutes | 10 minutes |
| Digoxin | 21 minutes | 18 minutes |
| Anti-HBc II | 27 minutes | 29 minutes |

Calculations relating to the SPT for a given test may be performed on the basis that the tests in a request have interaction effects $(\ldots + u_{1,2} T_1 T_2 + \ldots)$ on one another. The non-linear contributions may include vents such as time intervals for maintaining an analyzer to be used according to the test request, reagent consumption, etc, which may be laboratory specific and form a part of laboratory-specific historic data. The model itself could also be a GLM (generalized linear model) instead of a linear model. The formula in such a case may take the form $E(SPT) = \mu = h(u_0 + u_1 T_1 + \cdots)$. After identifying the form of the model, the parameters are fitted using historical data to obtain optimized values for all $u$. Using this, the SPT for a single sample can be obtained, where the request for the sample includes a certain combination of test to be performed.

The SPT for a single sample is then used to compute a total wait time, $W$, for a given set of multiple samples which are currently waiting in line to be processed by a given laboratory. This takes into account that there can be parallel executions (defined by the number of instruments and the number of tests that instrument can carry out in parallel).

An upper bound of the total waiting time, assuming no parallelization, is:

$$W \leq \sum_{i=1}^{n} SPT_i = W_{max}$$

A lower bound of the total waiting time would be assume perfect parallelization of each test:

$$W \geq nu_0 + \frac{\Sigma_{i=1}^{n} u_1 T_{i1}}{\#Parallel1} + \frac{\Sigma_{i=1}^{n} u_2 T_{i2}}{\#Parallel2} + \cdots = nu_0 + u_1 \frac{\Sigma_{i=1}^{n} T_{i1}}{\#Parallel1} + u_2 \frac{\Sigma_{i=1}^{n} T_{i2}}{\#Parallel2} + \cdots = W_{min} \qquad (1)$$

The values for u are estimated from historical data (e.g., as averages or from a machine-learning approach). It can be assumed that some values for $u$ are lab independent, however lab dependent values for $u$ may also be estimated if desired.

For the total workload then of a laboratory, all samples at that laboratory are combined which are: (i) actively being processed; (ii) queued to be processed next; and (iii) announced and in delivery. $W_{min}$ is then calculated according to equation (1) above. The number can be understood as: if a new sample request was received now, it would need to wait in the queue for this laboratory for at least $W_{min}$ to be processed. As discussed below, the overview of the waiting times for each laboratory can be collected and presented in a dashboard.

Table 2 below shows an example of the data used to calculate $W_{min}$. It can be described as a list of samples and their assigned requests, indications of those being actively processed, queued, or announced.

*Table 2*

| EventDateTime | EventType | Sample | Tests | Lab & Layout |
|---|---|---|---|---|
| 2023-06-06 0800 | Received | 2as1s15as | GLUC3, TSH | Lab X (2x TSH,...) |
| 2023-06-06 0800 | Received | das25ad617 | TSH, GLUC1, GLUC2, GLUC3 | Lab X (2x TSH,...) |
| 2023-06-06 1100 | Processed | 99df315aff | TSH, BSW, QVC, RTL | Lab Y (5x BSW, ...) |

This input data can be used both as a training set to obtain the complexity factors, and also as subsequent input data to obtain the total waiting time. In an example, the output of this stage is a table such as shown in Table 3:

*Table 3*

| $W_{min}$ | Lab |
|---|---|
| 32.4 hours | Lab X |
| 71 hours | Lab Y |
| 10 hours | Lab Z |

Further, the request can specify which of a plurality of laboratories the analytical test(s) are to be performed and can further specify a desired time to result (DTR) for receiving the results of the analytical test(s). The desired time to result can either be defined by the requester of the test (e.g., healthcare professional) or by a service level agreement. There are three components which make up the total time to result of a packaged sample: the transport time from the sample's origination point (e.g., clinic) to the laboratory ($T_{transport}$), the time during which the laboratory is processing samples already on-site and in the queue ($W_{min,lab}$), and the time it takes to process the sample itself ($W_{min,package}$):

$$T_{total} = T_{transport} + W_{min,lab} + W_{min,package} \qquad (2)$$

$W_{min,lab}$ is determined by formula (1). By analyzing the historical data (and/or, for example, geo-location) the time it takes to transport the sample from its origination point to a given laboratory, $T_{transport,}$ can be estimated. This is laboratory specific. $W_{min,package}$ can then be estimated by utilising equation (1) for the case where there is one sample to be processed (the sample in question). $T_{total}$ is therefore laboratory specific and can be used to access which laboratory would be able to process the sample the fastest.

Table 4 is an example of input data used during this process. In this example, a number of samples are placed into a single package:

*Table 4*

| EventDateTime | Package | Sample | Test | Sender | Labs | DTR |
|---|---|---|---|---|---|---|
| 2023-06-06 0800 | 234803 | 2as1s15as, das25ad617, ... | GLUC3, Ca, ... | Physician A | Lab X, Lab A, Lab Z, ... | 2023-06-07 0800 |
| 2023-06-06 0800 | 092309 | wdr25ad617, 5as1s15as, ... | TSH, GLUC3, ... | Physician B | Lab X, Lab Z, ... | 2023-06-07 0800 |
| 2023-06-06 1100 | 902302 | zdr25ad617, 5qs1s15as, ... | GLUC3, TSH, Ca, ... | Physician A | Lab X, Lab B, ... | 2023-06-07 1100 |

The output of this processing is a $T_{total}$ for each package (and so for its corresponding samples) for all of the laboratories to which they can be sent, as shown in Table 5:

*Table 5*

| Package | Lab | $T_{total}$ |
|---|---|---|
| 234803 | Lab X | 18 hours |
| 092309 | Lab X | 20 hours |
| 902302 | Lab X | 12 hours |
| 234803 | Lab Z | 10 hours |
| 092309 | Lab Z | 9 hours |

After the total time has been estimated for all of the laboratories, as determined in step S103, the method moves to step S104 where a routing action is performed.

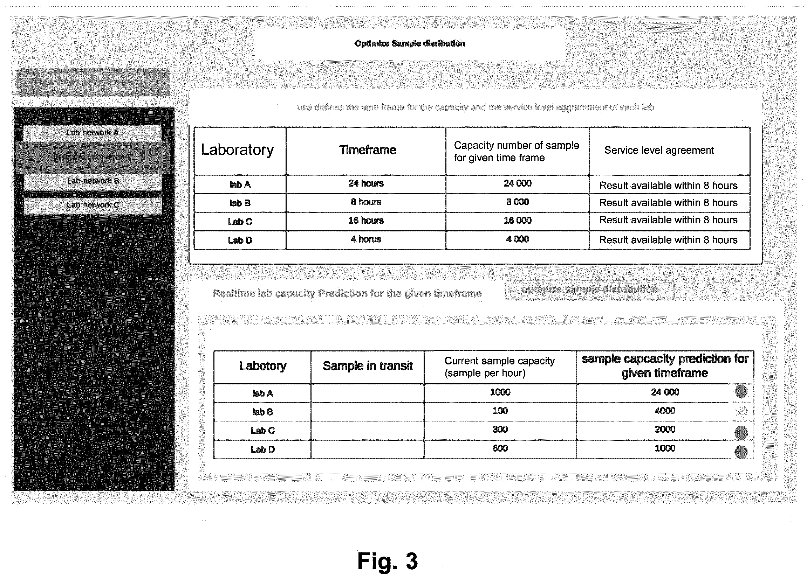

For example, the routing action can involve optimizing the sample distribution to the laboratory automatically based on the sample processing waiting time prediction, a sample distribution algorithm, communication with the one or more laboratories, and monitoring applications monitoring couriers transporting the samples. The samples are distributed based on the lab capacity within the network. Further, lab managers and/or directors are able to use a web application in order to obtain an overview of one or more current lab capacities (derived from the sample processing waiting time predictions). This is shown, for example, in Figure 3.

In other examples, the routing action may include recommending to a patient via a patient portal (not shown, by connected to network 120) that they visit one or more medical centres or one or more of the laboratories for the sample to be taken. Recommending the medical centre to visit includes determining, from an electronic health record, a time of a follow-up medical appointment and recommending the one or more medical centres, or laboratories, where the total time, $T_{total}$, of the or each laboratory associated with the respective medical centre indicates the sample will be processed ahead of the follow-up medical appointment.

The user, for example the lab manager and/or director, has access to the web application and is able to define the expected capacity of each laboratory within their network. Capacity is defined as the number of samples that the laboratory is expected to be able to process for a given time. Therefore, the input of the user for each laboratory in the network will be the number of samples that should be processed for the given timeframe. In order to promote an optimized sample distribution, the user also defines the service level agreement which defines the expected time that the test result should be available. As shown in Figure 3, the SLA for Labs A - D is 8 hours.

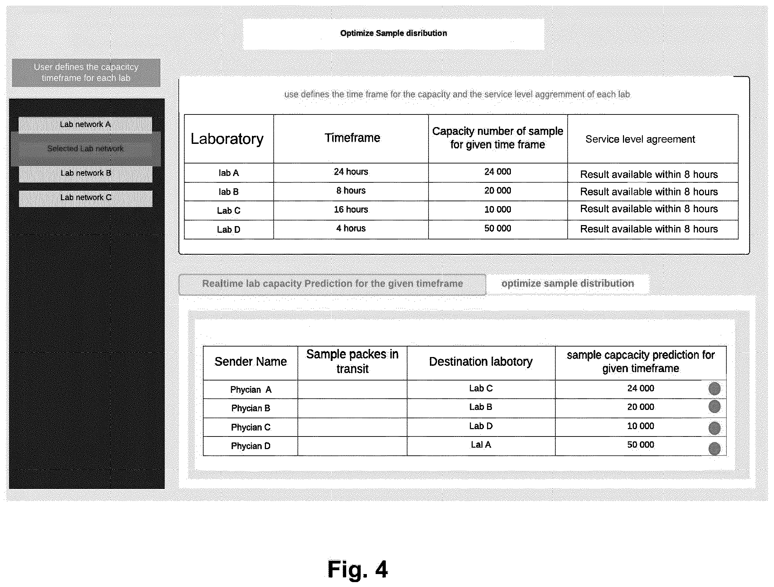

In real time, the current capacity (the sample processing wait time) and the planned samples in transit are monitored in real time. This information can be used to predict the sample capacity for each given time frame. The result of the optimization is shown in Figure 4, where incoming samples have been distributed to the relevant lab within the network by considering the time the test result availability of each laboratory.

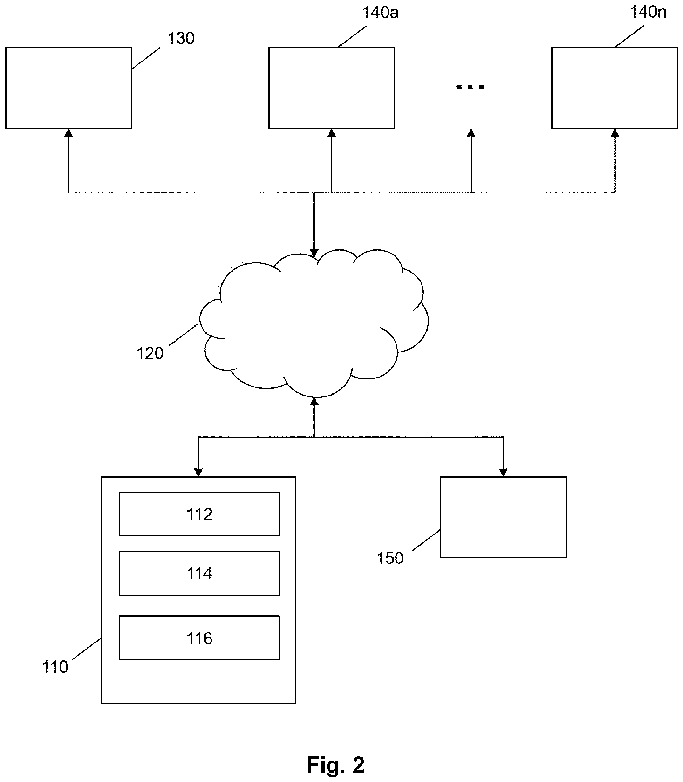

Figure 2 is an example of a system according to an aspect of the present disclosure. The system comprises a computer 110, which includes a request processing module 112; a laboratory capacity analysis module 114; and a sample distribution module 116. The computer 110, which may be a virtual computer running in a cloud environment, is connected via network 120 to the following: a sample request ingress device 130; a plurality of laboratory data sources 140a - 140n; and an output device 150. The sample request ingress device 130 may be, for example, a clinician's terminal used to generate requests for analytical tests as discussed previously. Alternatively, it may be a gateway which receives such requests, and forwards them to the request processing module 112. That is, the request processing module 112 may receive a request for an analytical test directly from a clinician or via a gateway and so performs the step S101 discussed above with relation to Figure 1.

The laboratory data sources, which may be laboratory information systems, healthcare information systems where the laboratory is within a hospital or which may be provided as middleware within a laboratory computer network, generate or obtain the data required for the laboratory capacity analysis module 114 to perform the methods as discussed previously. That is, they may generate or obtain historical data which can be used in the methods discussed herein (e.g., the derivation of each complexity factor u). Further, the laboratory data sources provide the data used to make live predictions. This can include, for example, the current or scheduled availability statuses of the various analytical instruments within the laboratories, as well as reagent levels, required quality control testing runs, and/or maintenance schedules.

In a variant example, not shown here, each laboratory data sources may include or be connected to a local version of the laboratory capacity analysis module 114 (and so computer 110 omits it). In such examples, each local laboratory capacity analysis module reports to the sample distribution module the estimated total time $T_{total}$ for the lab(s) to which it relates. For example, some laboratory information systems may manage a network of laboratories and so would report a $T_{total}$ for each of the laboratories within its network.

The sample distribution module 116 receives both the request for the analytical test and the reported $T_{total}$ for the various laboratories, and performs the routing actions as discussed above. For example, it may route the sample to a specific laboratory for processing so as to ensure that it is processed in time for the result to be reported under an SLA. This can include automatically redirecting a sample from a laboratory which was specified in the request to a different laboratory, where it is determined that the specified laboratory would be unable to process the same within the SLA.

The output device 150 is also connected to the laboratory analysis module 114 (or to each local laboratory analysis module as the case may be). It can, for example, provide the web interface discussed above allowing lab managers and/or directors to access the current lab capacity information and so take corrective action where required. In this example the output device 150 is discrete from the computer 110. However, in some examples it may be incorporated within the computer as an additional module thereof.

The method disclosed herein may be cloud based, and make use of edge devices and their corresponding functionality and connectivity to collect real time streaming events from one or more of the laboratories. The cloud based functionality may also connect to a sample monitoring application, the application communicating in real time via a standard protocol to an end point of the cloud provider. The method monitors sample processing of each laboratory, and samples being collected and in transition to the laboratories. The stream of events is captured via cloud provided stream injection services which enable the connection of a very large number of laboratories (e.g., thousands) in a secure and reliable manner. The cloud injection service can be used to push events into an event stream storage, where the necessary information can be extracted, transformed, and loaded for processing in real time. During processing, features in the data can be extracted from the laboratories and used for a daily (weekly, hourly, or other time interval based) sample processing waiting time prediction for each laboratory. The data features extracted from the sample monitoring application can be used in a sample distribution algorithm as discussed herein.

The events stream of the sample processing of each laboratory and sample monitoring application can be used to trigger an algorithm that is capable of predicting the capacity of the laboratory in real time. The, for example, daily prediction indicates how many samples that laboratory is expected to be capable of processing within a given time (e.g., a day). In the case that the prediction indicates that the laboratory is reaching its limit (e.g., through impact on key performance indexes such as turnaround-time), an algorithm may automatically be triggered that calculates an optimized distribution of samples across the laboratory network and so the sample load is balanced accordingly. The optimized sample distribution can be communicated to each sample monitoring application, so that the distribution can take place. The lab network director / manager will also be able to access the sample distribution via a web application, and further refine it if needed.

The systems and method disclosed herein can be used in the context of telemedicine, whereby it recommends a laboratory for sample collection so that the availability of the sample result matches the patient's next visit. Proposed is a computer-implemented method for routing biological samples, for example allocating them to a laboratory for processing, the method comprising:

receiving a request for an analytical test, the request defining one or more analytical tests to be performed on a biological sample;
estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and

performing a routing action related to the biological sample on the basis of the respective total times.

Also proposed is a computer-implemented method for telemedicine sample analysis and/or processing, the method comprising:

receiving a request for an analytical test, the request defining one or more analytical tests to be performed on a biological sample;

estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and

performing a routing action related to the biological sample on the basis of the respective total times.

Further proposed is a computer-implemented method for re-allocating one or more biological samples within a laboratory, the method comprising:

receiving a request for an analytical test, the request defining one or more analytical tests to be performed on a biological sample;

estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and

performing a routing action related to the biological sample on the basis of the respective total times.

Further proposed is a computer-implemented method for estimating a total time $T_{total}$ for a biological sample to be processed, the method comprising:

receiving a request for an analytical test, the request defining one or more analytical tests to be performed on a biological sample;

estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and

estimating the total time $T_{total}$ of the biological sample on the basis of the respective total times.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A computer-implemented method, the method comprising:

   receiving a request for an analytical test, the request defining one or more analytical tests to be performed on a biological sample;

   estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and

   performing a routing action related to the biological sample on the basis of the respective total times.

2. The computer-implemented method of claim 1, wherein using the model to estimate the respective total time, $T_{total}$, for each laboratory includes estimating, for each of a plurality of pending biological samples, a pending sample processing time, $SPT$, indicative of the time taken for the pending biological sample to be tested; wherein each pending sample processing time, $SPT$, is determined on the basis of the one or more complexity factors.

3. The computer-implemented method of claim 2, wherein estimating the respective total time, $T_{total}$, for each laboratory includes estimating a total pending samples processing time, $W$, on the basis of the pending sample processing times, $SPT$.

4. The computer-implemented method of claim 3, wherein estimating the total pending samples processing time, $W$, includes determining whether any of the analytical tests for the pending samples can be performed in parallel and adjusting the calculation of the total pending samples processing time, $W$, based on this determination.

5. The computer-implemented method of any of claims 2-4, wherein the sample processing time, $SPT$, for a given pending sample, $i$, is estimated using the model, wherein the model is a regression model of the form:

$$SPT_i = \sum_j^p u_j T_{ij},$$

where $u_j$ is one of the complexity factors corresponding to analytical test $j$ to be performed on the sample, $i$, the variable $T_{ij}$ is either 0 or 1 dependant on whether the test $j$ is to be performed on the sample $i$, and $p$ is the total number of tests to be performed on the respective pending sample, the complexity factors $u_j$ being determined based on the historical testing data.

6. The computer-implemented method of any preceding claim, wherein estimating the respective total time, $T_{total}$, further includes estimating a time taken for the sample to arrive at the respective laboratory, $T_{transport}$, and/or the time taken to process the biological sample at that respective laboratory $W_{min,package}$.

7. The computer-implemented method of any preceding claim, wherein the method further includes providing the estimated respective total time $T_{total}$, for each of the plurality of laboratories on a dashboard.

8. The computer-implemented method of any preceding claim, wherein the routing action comprises routing the sample to a laboratory of the plurality of laboratories selected on the basis of the plurality of estimated total times.

9. The computer-implemented method of any preceding claim, wherein the request also specifies one or more laboratories at which the analytical test can be performed, wherein the laboratory is further selected on the basis of the specified laboratories.

10. The computer-implemented method of any preceding claim, wherein the request also specifies a desired time to result, DTR, for receiving results of the one or more analytical tests, and wherein the routing action comprises selecting a laboratory from a list of laboratories which have a total time, $T_{total}$, which fulfils the desired time to result.

11. The computer-implemented method of claim 10 wherein:

the request includes a specified laboratory,
the method comprises comparing the DTR with the respective total time, $T_{total}$, for the specified laboratory, and, if the respective total time, $T_{total}$, is longer than the desired time to result, determining an alternative laboratory having a $T_{total}$, shorter than the DTR.

12. The computer-implemented method of any preceding claim, wherein the routing action comprises recommending to a patient one or more medical centres to visit for sample collection.

13. The computer-implemented method of claim 12, wherein recommending the medical centre to visit includes determining, from an electronic health record, a time of a follow-up medical appointment and recommending the one or more medical centres where the total time, $T_{time}$, of the or each laboratory associated with the respective medical centre indicates the sample will be processed ahead of the follow-up medical appointment.

14. A computer, comprising a processor and memory, the memory containing machine-executable instructions which when executed on the processor cause the processor to:

receive a request for an analytical test, the request defining one or more analytical tests to be performed on a sample;
estimate, for at least one of plurality of laboratories, a respective total time, $T_{total}$, to process the sample by the respective laboratory, wherein estimating the respective total time is performed using a model which includes one or more complexity factors based on the complexity of one or more analytical tests to be performed by the respective laboratory, the complexity factor being derived from historical testing data; and
perform a routing action related to the biological sample on the basis of the respective total times.

15. A system for allocating biological samples to laboratories for analytical tests, the system comprising:

a request processing module, configured to receive a request for an analytical test, the request defining one or more analytical tests to be performed on a biological sample;
a laboratory capacity analysis module, configured to estimate for each of a plurality of a plurality of laboratories, a respective total time, $T_{total}$, to process the analytical tests to be performed on the biological sample by the respective laboratory, wherein estimating the total time is performed using a model which includes a complexity factory based on the complexity of the one or more analytical tests to be performed, the complexity factor being derived from historical testing data; and
a sample distribution module, configured to determine a routing action related to the biological sample on the basis of the respective total times.

**Amended claims in accordance with Rule 137(2) EPC.**

1. A computer-implemented method, the method comprising:

   receiving a request for an analytical test, the request defining one or more analytical tests to be performed on a biological sample;
   estimating, for each of a plurality of laboratories, a respective total time, $T_{total}$, to process the biological sample by the respective laboratory, wherein estimating the total time is performed using a model which includes one or more complexity factors indicating a complexity of one or more analytical tests to be performed by the respective laboratory, the complexity factors being derived from historical testing data; and
   performing a routing action related to the biological sample on the basis of the respective total times.

2. The computer-implemented method of claim 1, wherein using the model to estimate the respective total time, $T_{total}$, for each laboratory includes estimating, for each of a plurality of pending biological samples, a pending sample processing time, $SPT$, indicative of the time taken for the pending biological sample to be tested; wherein each pending sample processing time, $SPT$, is determined on the basis of the one or more complexity factors.

3. The computer-implemented method of claim 2, wherein estimating the respective total time, $T_{total}$, for each laboratory includes estimating a total pending samples processing time, $W$, on the basis of the pending sample processing times, $SPT$.

4. The computer-implemented method of claim 3, wherein estimating the total pending samples processing time, W, includes determining whether any of the analytical tests for the pending samples can be performed in parallel and adjusting the calculation of the total pending samples processing time, W, based on this determination.

5. The computer-implemented method of any of claims 2 - 4, wherein the sample processing time, $SPT$, for a given pending sample, i, is estimated using the model, wherein the model is a regression model of the form:

$$SPT_i = \sum_j^p u_j T_{ij} \, ,$$

   where $u_j$ is one of the complexity factors corresponding to analytical test j to be performed on the sample, i, the variable $T_{ij}$ is either 0 or 1 dependant on whether the test j is to be performed on the sample i, and p is the total number of tests to be performed on the respective pending sample, the complexity factors $u_j$ being determined based on the historical testing data.

6. The computer-implemented method of any preceding claim, wherein estimating the respective total time, $T_{total}$, further includes estimating a time taken for the sample to arrive at the respective laboratory, $T_{transport}$, and/or the time taken to process the biological sample at that respective laboratory $W_{min,pakage}$.

7. The computer-implemented method of any preceding claim, wherein the method further includes providing the estimated respective total time $T_{total}$, for each of the plurality of laboratories on a dashboard.

8. The computer-implemented method of any preceding claim, wherein the routing action comprises routing the sample to a laboratory of the plurality of laboratories selected on the basis of the plurality of estimated total times.

9. The computer-implemented method of any preceding claim, wherein the request also specifies one or more laboratories at which the analytical test can be performed, wherein the laboratory is further selected on the basis of the specified laboratories.

10. The computer-implemented method of any preceding claim, wherein the request also specifies a desired time to result, DTR, for receiving results of the one or more analytical tests, and wherein the routing action comprises selecting a laboratory from a list of laboratories which have a total time, $T_{total}$, which fulfils the desired time to result.

11. The computer-implemented method of claim 10 wherein:

    the request includes a specified laboratory,
    the method comprises comparing the DTR with the respective total time, $T_{total}$, for the specified laboratory, and,

if the respective total time, $T_{total}$, is longer than the desired time to result, determining an alternative laboratory having a $T_{total}$, shorter than the DTR.

12. The computer-implemented method of any preceding claim, wherein the routing action comprises recommending to a patient one or more medical centres to visit for sample collection.

13. The computer-implemented method of claim 12, wherein recommending the medical centre to visit includes determining, from an electronic health record, a time of a follow-up medical appointment and recommending the one or more medical centres where the total time, $T_{time}$, of the or each laboratory associated with the respective medical centre indicates the sample will be processed ahead of the follow-up medical appointment.

14. A computer readable medium storing a computer program, comprising code, which when run on a computer, cause the computer to perform the computer-implemented method of any preceding claim.

15. A system for allocating biological samples to laboratories for analytical tests, the system comprising:

a request processing module, configured to receive a request for an analytical test, the request defining one or more analytical tests to be performed on a biological sample;

a laboratory capacity analysis module, configured to estimate for each of a plurality of a plurality of laboratories, a respective total time, $T_{total}$, to process the analytical tests to be performed on the biological sample by the respective laboratory, wherein estimating the total time is performed using a model which includes a complexity factory based on the complexity of the one or more analytical tests to be performed, the complexity factor being derived from historical testing data; and

a sample distribution module, configured to determine a routing action related to the biological sample on the basis of the respective total times.

S101

Receive request

S102

Estimate total time
for laboratory *n*

S103

Calculated for all
laboratories?

No

Yes

S104

Perform routing
action

**Fig. 1**

**Fig. 2**

**Optimize Sample distribution**

use defines the time frame for the capacity and the service level agreement of each lab

| Laboratory | Timeframe | Capacity number of sample for given time frame | Service level agreement |
|---|---|---|---|
| lab A | 24 hours | 24 000 | Result available within 8 hours |
| lab B | 8 hours | 8 000 | Result available within 8 hours |
| Lab C | 16 hours | 16 000 | Result available within 8 hours |
| Lab D | 4 horus | 4 000 | Result available within 8 hours |

User defines the capacity timeframe for each lab

Lab network A

Selected Lab network

Lab network B

Lab network C

Realtime lab capacity Prediction for the given timeframe

optimize sample distribution

| Labotory | Sample in transit | Current sample capacity (sample per hour) | sample capacity prediction for given timeframe |
|---|---|---|---|
| lab A | | 1000 | 24 000 |
| lab B | | 100 | 4000 |
| Lab C | | 300 | 2000 |
| Lab D | | 600 | 1000 |

# Fig. 3

Optimize Sample disribution

Lab network A

Selected Lab network

Lab network B

Lab network C

use defines the time frame for the capacity and the service level aggremment of each lab

| Laboratory | Timeframe | Capacity number of sample for given time frame | Service level agreement |
|---|---|---|---|
| lab A | 24 hours | 24 000 | Result available within 8 hours |
| lab B | 8 hours | 20 000 | Result available within 8 hours |
| Lab C | 16 hours | 10 000 | Result available within 8 hours |
| Lab D | 4 horus | 50 000 | Result available within 8 hours |

Realtime lab capacity Prediction for the given timeframe | optimize sample distribution

| Sender Name | Sample packes in transit | Destination labotory | sample capcacity prediction for given timeframe | |
|---|---|---|---|---|
| Phycian A | | Lab C | 24 000 | ● |
| Phycian B | | Lab B | 20 000 | ● |
| Phycian C | | Lab D | 10 000 | ● |
| Phycian D | | Lal A | 50 000 | ● |

# Fig. 4

EP 4 621 795 A1

Europäisches
Patentamt

European
Patent Office

Office européen
des brevets

# EUROPEAN SEARCH REPORT

**Application Number**

EP 24 16 4662

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/328144 A1 (CORMANO BEL HECTOR [ES] ET AL) 13 October 2022 (2022-10-13) * The whole document, in particular: Paragraphs [0006], [0016], [0025] - [0027], [0030], [0033], [0037], [0060]. * | 1-15 | INV. G16H40/20 G16H10/40 |
| X | US 2013/197943 A1 (CONLIN PAUL [US] ET AL) 1 August 2013 (2013-08-01) * The whole document, in particular: Paragraphs [0005], [0006], [0051] - [0054], [0067], [0074], [0084] - [0097]. * | 1-15 | |
| X | US 2007/294103 A1 (AHMAD AZMAT Z [US] ET AL) 20 December 2007 (2007-12-20) * The whole document, in particular: Paragraphs [0009], [0033] - [0043]. * | 1-15 | |

| | |
|---|---|
| | **TECHNICAL FIELDS SEARCHED (IPC)** |
| | G16H |

The present search report has been drawn up for all claims

| Place of search | Date of completion of the search | Examiner |
|---|---|---|
| Munich | 26 July 2024 | Nagele, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 16 4662

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2024

| Patent document cited in search report | | Publication date | Patent family member(s) | | Publication date |
|---|---|---|---|---|---|
| US 2022328144 | A1 | 13-10-2022 | CN | 115301310 A | 08-11-2022 |
| | | | EP | 4071686 A1 | 12-10-2022 |
| | | | JP | 2022161868 A | 21-10-2022 |
| | | | US | 2022328144 A1 | 13-10-2022 |
| US 2013197943 | A1 | 01-08-2013 | NONE | | |
| US 2007294103 | A1 | 20-12-2007 | NONE | | |

EPO FORM P0459

For more details about this annex : see Official Journal of the European Patent Office, No. 12/82